# EUROPEAN PATENT APPLICATION

(11) **EP 4 424 777 A1**
(43) Date of publication of application: **04.09.2024**
(21) Application number: 22887184.4
(22) Date of filing: 28.10.2022
(51) Int. Cl.: C08L 101/04, A61F 13/15, A61F 13/53, A61L 15/18, A61L 15/22, A61L 15/24, A61L 15/42, A61L 15/46, C08K 3/04

(54) **WATER-ABSORBENT RESIN COMPOSITION, ABSORBER, AND WATER-ABSORBENT ARTICLE**

(30) Priority: 29.10.2021 JP 2021178142
(71) Applicant: Sumitomo Seika Chemicals Co., Ltd., Kako-gun, Hyogo 675-0145 (JP)
(72) Inventor: YAMAMOTO, Tomoe, Himeji-shi, Hyogo 672-8076 (JP)
(74) Representative: Mewburn Ellis LLP
(86) International application number: PCT/JP2022/040440
(87) International publication number: WO 2023/074859

(57) **Abstract**

Provided is a water-absorbent resin composition comprising water-absorbent resin particles and an activated carbon, which has an excellent deodorizing effect. A water-absorbent resin composition, comprising water-absorbent resin particles and an activated carbon disposed on surface of the water-absorbent resin particles, wherein when, while stirring 50 g of physiological saline at 24.9°C at 600 rpm in a 300 mL thermally insulated container, 10 g of the water-absorbent resin composition is added and swollen, the water-absorbent resin composition has a heat release rate of 0.10°C/sec or more and 1.00°C/sec or less until temperature increases by 2°C.

## Description

### Technical Field

The present invention relates to a water-absorbent resin composition, an absorbent material, and an absorbent article; and more particularly relates to a water-absorbent resin composition that constitutes an absorbent material suitably used for hygienic materials, such as disposable diapers, sanitary napkins, and incontinence pads, and to a method for producing the water-absorbent resin composition.

### Background Art

In recent years, water-absorbent resins have been widely used in the field of hygienic materials, such as disposable diapers, sanitary napkins, and incontinence pads.

As such water-absorbent resins, crosslinked products of partially neutralized salt polymers of acrylic acid have been proposed as preferable water-absorbent resins, because they have many advantages. For example, these water-absorbent resins have good water-absorption capacity; acrylic acid used as a raw material is readily industrially available, and thus, they can be produced at low cost with uniform quality; additionally, they are resistant to decomposition or degradation.

An absorbent article, such as a disposable diaper, a sanitary napkin, or an incontinence pad, is composed of an absorbent material that absorbs and retains a body liquid excreted from the body, such as urine or menstrual blood , the absorbent material being positioned mainly in a central portion, a liquid-permeable front sheet (top sheet) positioned on the side of the absorbent article that is brought into contact with the body, and a liquid-impermeable rear sheet (back sheet) positioned opposite to the side that is brought into contact with the body. The absorbent material is typically composed of hydrophilic fibers, such as pulp, and a water-absorbent resin.

When such an absorbent material is used in, for example, a hygienic material, an unpleasant odor such as ammonia or acetaldehyde may be produced from the absorbent material that has absorbed a body fluid, particularly urine, blood, sweat, or the like.

One known method for reducing such an unpleasant odor is, for example, to add an organic amine compound such as a hydrazide compound to a water-absorbent resin as an adsorbent for aldehyde compounds (see Patent Literature 1).

### Citation List

### Patent Literature

Patent Literature 1: JP-A-2001-323155

### Summary of Invention

### Technical Problem

As described above, one known method for reducing the unpleasant odor produced from the absorbent material is, for example, to use an organic amine compound as an adsorbent for acetaldehyde.

On the other hand, activated carbons are known as adsorbents having an excellent deodorizing effect against a wide range of odors. Thus, the present inventor combined water-absorbent resin particles with an activated carbon in an attempt to impart the deodorizing effect to the absorbent material.

It is a main object of the present invention to provide a water-absorbent resin composition comprising water-absorbent resin particles and an activated carbon, which has an excellent deodorizing effect.

### Solution to Problem

The present inventor has conducted extensive research to solve the aforementioned problem. As a result, the inventor has found that when a water-absorbent resin composition comprising a combination of water-absorbent resin particles and an activated carbon has an increased heat release rate during absorption of physiological saline, the water-absorbent resin composition can exhibit an improved deodorizing effect. The present invention has been accomplished as a result of further research based on these findings.

In summary, the present invention provides aspects of the invention comprising the following features:
Item 1. A water-absorbent resin composition, comprising water-absorbent resin particles and an activated carbon disposed on surfaces of the water-absorbent resin particles,
   wherein when, while stirring 50 g of physiological saline at 24.9°C at 600 rpm in a 300 mL thermally insulated container, 10 g of the water-absorbent resin composition is added and swollen, the water-absorbent resin composition has a heat release rate of 0.10°C/sec or more and 1.00°C/sec or less until temperature increases by 2°C.
Item 2. The water-absorbent resin composition according to item 1, wherein the activated carbon content is 0.05% by mass or more and 1% by mass or less.
Item 3. The water-absorbent resin composition according to item 1 or 2, wherein the activated carbon has a median particle size of 1 µm or more and 500 µm or less.
Item 4. An absorbent material comprising the water-absorbent resin composition according to any one of items 1 to 3.
Item 5. An absorbent article comprising the absorbent material according to item 4.

### Advantageous Effects of Invention

According to the present invention, it is possible to provide a water-absorbent resin composition comprising water-absorbent resin particles and an activated carbon, which has an excellent deodorizing effect. Furthermore, according to the present invention, it is also possible to provide an absorbent material obtained using the water-absorbent resin composition, and an absorbent article obtained using the water-absorbent resin composition.

### Brief Description of Drawings

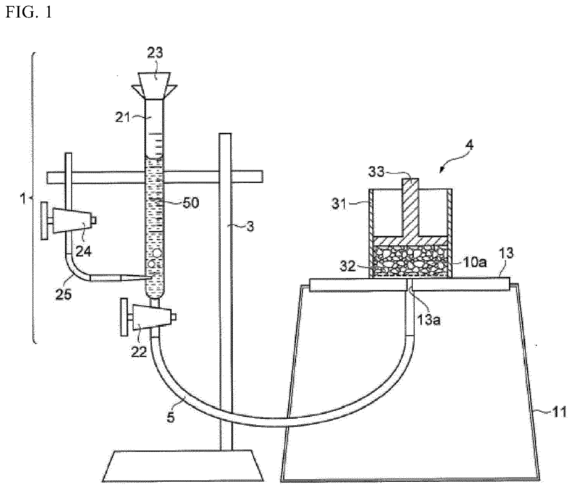

Fig. 1 is a schematic diagram of a measurement apparatus for physiological saline absorption amount under a load of 4.14 kPa.

### Description of Embodiments

As used herein, the term "comprising" includes "consisting essentially of" and "consisting of". As used herein, the term "(meth)acrylic" refers to "acrylic or methacrylic", and the term "(meth)acrylate" refers to "acrylate or methacrylate". As used herein, the term "water-soluble" refers to having a water solubility of 5% by mass or more at 25°C.

As used herein, values connected with "to" refer to the numerical range including the values before and after "to" as the lower and upper limits. When a plurality of lower limits and a plurality of upper limits are mentioned separately, any lower limit and any upper limit may be selected and connected with "to".

### 1.Water-Absorbent Resin Composition

The water-absorbent resin composition of the present invention is a water-absorbent resin composition comprising water-absorbent resin particles and an activated carbon disposed on surfaces of the water-absorbent resin particles, wherein when, while stirring 50 g of physiological saline at 24.9°C at 600 rpm in a 300 mL thermally insulated container, 10 g of the water-absorbent resin composition is added and swollen, the water-absorbent resin composition has a heat release rate of 0.10°C/sec or more and 1.00°C/sec or less until temperature increases by 2°C. The water-absorbent resin composition having these features exhibits an excellent deodorizing effect. The water-absorbent resin composition of the present invention will be hereinafter described in detail.

In the water-absorbent resin composition of the present invention, when, while stirring 50 g of physiological saline at 24.9°C at 600 rpm in a 300 mL thermally insulated container, 10 g of the water-absorbent resin composition is added and swollen, the water-absorbent resin composition has a heat release rate of 0.10°C/sec or more and 1.00°C/sec or less until temperature increases by 2°C. In the water-absorbent resin composition of the present invention, which is a water-absorbent resin composition comprising a combination of water-absorbent resin particles and an activated carbon, the heat release rate during absorption of physiological saline is adjusted to this high value, leading to an excellent deodorizing effect.

Examples of means for efficiently increasing the heat release rate in the water-absorbent resin composition of the present invention include using water-absorbent resin particles having an appropriate median particle size; using water-absorbent resin particles with a large specific surface area; using water-absorbent resin particles having increased hydrophilicity on the surfaces of the water-absorbent resin particles and thus having a high water-absorption rate; using surface-crosslinked water-absorbent resin particles; and using an activated carbon with a large specific surface area. These means may be used in combination according to the purpose.

In view of more satisfactorily achieving the effects of the present invention, the heat release rate is preferably 0.10°C/sec or more, more preferably 0.12°C /sec or more, still more preferably 0.14°C/sec or more, and even more preferably 0.20°C/sec or more. On the other hand, the heat release rate is preferably 1.00°C/sec or less, more preferably 0.80°C/sec or less, still more preferably 0.60°C/sec or less, and even more preferably 0.50°C/sec or less. The range of the heat release rate is preferably from 0.10 to 1.00°C/sec, more preferably from 0.12 to 0.80°C/sec, still more preferably from 0.14 to 0.6°C/sec, and even more preferably from 0.20 to 0.50°C/sec.

### (Activated Carbon)

In view of more satisfactorily achieving the effects of the present invention, the activated carbon preferably has a median particle size of 1 µm or more, more preferably 5 µm or more, still more preferably 10 µm or more. On the other hand, the median particle size is preferably 500 µm or less, more preferably 250 µm or less, still more preferably 100 µm or less, even more preferably 50 µm or less, and particularly preferably 45 µm or less. Preferred ranges include from 1 to 500 µm and from 5 to 50 µm.

The median particle size (D50 (median size), volume-based) of the activated carbon can be measured using a laser diffraction particle size distribution analyzer; specifically, it is the value as measured by the method described in the Examples section.

In view of more satisfactorily achieving the effects of the present invention, the activated carbon preferably has a shape such as a crushed shape or a cylindrical shape, more preferably a crushed shape.

In view of more satisfactorily achieving the effects of the present invention, the activated carbon preferably has a BET specific surface area of 100 m²/g or more, more preferably 1000 m²/g or more. On the other hand, the BET specific surface area is preferably 3000 m²/g or less, and more preferably 2000 m²/g or less. Preferred ranges include from 100 to 3000 m²/g and from 1000 to 2000 m²/g.

The BET specific surface area of the activated carbon can be measured using a specific surface area analyzer; specifically, it is the value as measured by the method described in the Examples section.

In view of more satisfactorily achieving the effects of the present invention, the activated carbon is preferably an activated carbon having a polar functional group (hydrophilic functional group) on the surface (i.e., a hydrophilic activated carbon). Examples of polar functional groups include hydroxy, carboxy, and phenol groups. Activated carbons having polar functional groups on the surface are commercially available as, for example, activated carbons for liquid phase applications and activated carbons for water treatment.

The activated carbon may be produced from, for example, coconut shells, infusibilized or carbonized organic materials, and infusible resins such as phenol resins. Examples of the organic materials include polyacrylonitrile, pitch, polyvinyl alcohol, and cellulose. Among these, the activated carbon is preferably produced from coconut shells or pitch (such as coal pitch or cellulose pitch).

In view of more satisfactorily achieving the effects of the present invention, the activated carbon content in the water-absorbent resin composition of the present invention is preferably 0.05% by mass or more, more preferably 0.07% by mass or more, and still more preferably 0.1% by mass or more, while it is preferably 10% by mass or less, more preferably 5% by mass or less, still more preferably 1% by mass or less, and even more preferably 0.5% by mass or less. Preferred ranges include from 0.05 to 10% by mass and from 0.1 to 1% by mass.

In view of more satisfactorily achieving the effects of the present invention, the activated carbon preferably has an iodine adsorption amount of 100 mg/g or more, more preferably 500 mg/g or more. On the other hand, the iodine adsorption amount is preferably 3000 mg/g or less, and more preferably 2000 mg/g or less. Preferred ranges include from 100 to 3000 mg/g and from 500 to 2000 mg/g.

As used herein, the iodine adsorption amount of the activated carbon is the value as measured according to JIS K 1474: 2014.

In view of more satisfactorily achieving the effects of the present invention, the activated carbon preferably has a loss on drying of 0.1% or more, more preferably 0.5% or more. On the other hand, the loss on drying is preferably 8% or less, and more preferably 5% or less. Preferred ranges include from 0.1 to 8% and from 0.5 to 5%.

As used herein, the loss on drying of the activated carbon is the value as measured according to JIS K 1474: 2014.

In view of more satisfactorily achieving the effects of the present invention, the activated carbon preferably has a pH of 3 or more, more preferably 4 or more, or even 5 or more. On the other hand, the pH is preferably 12 or less, and more preferably 11 or less. Preferred ranges include from 3 to 12, from 4 to 11, and from 5 to 11.

As used herein, the pH of the activated carbon is the value as measured according to JIS K 1474: 2014.

In the water-absorbent resin composition of the present invention, preferably, the activated carbon is disposed on surfaces of the water-absorbent resin particles (i.e., the activated carbon is present on surfaces of the water-absorbent resin particles). As described below, the activated carbon can be disposed on the surfaces of the water-absorbent resin particles by, for example, mixing the water-absorbent resin particles and the activated carbon in a solid state, so that the activated carbon adheres to the surfaces of the water-absorbent resin particles.

The water-absorbent resin contained in the water-absorbent resin composition of the present invention will be described in detail next.

### (Water-Absorbent Resin Particles)

The water-absorbent resin particles contained in the water-absorbent resin composition of the present invention are formed of a crosslinked product of a polymer of a water-soluble ethylenically unsaturated monomer, that is, a crosslinked polymer having a structural unit derived from a water-soluble ethylenically unsaturated monomer.

In view of more satisfactorily achieving the effects of the present invention, the water-absorbent resin particles preferably have a water-absorption rate, as measured by a Vortex method, of 60 seconds or less, more preferably 50 seconds or less, still more preferably 40 seconds or less. On the other hand, the water-absorption rate is preferably 1 second or more, more preferably 3 seconds or more, and still more preferably 10 seconds or more. Preferred ranges include from 1 to 60 seconds and from 3 to 40 seconds.

The water-absorption rate of the water-absorbent resin particles as measured by the Vortex method is the value as measured by the method described in the Examples section.

In view of more satisfactorily achieving the effects of the present invention, the water-absorbent resin particles preferably have a physiological saline retention amount of 20 g/g or more, more preferably 25 g/g or more, still more preferably 30 g/g or more. On the other hand, the physiological saline retention amount is preferably 60 g/g or less, more preferably 55 g/g or less, and still more preferably 50 g/g or less, for example.

In view of more satisfactorily achieving the effects of the present invention, the water-absorbent resin particles preferably have a physiological saline absorption amount under a load of 4.14 kPa of 5 mL/g or more, more preferably 10 mL/g or more, still more preferably 15 mL/g or more. On the other hand, the physiological saline absorption amount under a load of 4.14 kPa is preferably 40 mL/g or less, more preferably 35 mL/g or less, and still more preferably 30 mL/g or less. Preferred ranges include from 5 to 40 mL/g.

The physiological saline retention amount and the physiological saline absorption amount under a load of 4.14 kPa of the water-absorbent resin particles are each the value as measured by the method described in the Examples section.

In view of more satisfactorily achieving the effects of the present invention, the water-absorbent resin particles preferably have a BET specific surface area of 0.01 m²/g or more, more preferably 0.02 m²/g or more. On the other hand, the BET specific surface area is preferably 0.20 m²/g or less, and more preferably 0.150 m²/g or less. Preferred ranges include from 0.01 to 0.20 m²/g and from 0.02 to 0.15 m²/g.

The BET specific surface area of the water-absorbent resin particles can be measured using a specific surface area analyzer; specifically, it is the value as measured by the method described in the Examples section.

The water-absorbent resin is typically particulate. In view of satisfactorily achieving the effects of the present invention while avoiding local absorption in the absorbent article, the water-absorbent resin particles preferably have a median particle size of 150 µm or more, 200 µm or more, 240 µm or more, 260 µm or more, 280 µm or more, or 300 µm or more. On the other hand, in view of satisfactorily achieving the effects of the present invention while imparting a comfortable feel to the absorbent article, the median particle size is preferably 850 µm or less, 600 µm or less, 550 µm or less, 500 µm or less, 450 µm or less, or 400 µm or less. That is, the median particle size is preferably 150 to 850 µm, more preferably 200 to 600 µm, still more preferably 240 to 500 µm, even more preferably 280 to 450 µm, and still more preferably 300 to 400 µm. The water-absorbent resin composition of the present invention also preferably has a median particle size of 150 to 850 µm, more preferably 200 to 600 µm, still more preferably 240 to 500 µm, even more preferably 280 to 450 µm, and still more preferably 300 to 400 µm.

The water-absorbent resin particles may be in the form of particles each composed of a single particle, or in the form of particles (secondary particles) formed by agglomeration of fine particles (primary particles). Examples of the shape of the primary particles include a substantially spherical shape, a crushed indefinite shape, and a flat shape. When the water-absorbent resin particles are primary particles produced by reversed phase suspension polymerization, examples of the shape include a substantially spherical single-particle shape with a smooth surface shape, such as a spherical shape or an elliptical sphere shape.

The median particle size of the water-absorbent resin particles can be measured using JIS standard sieves; specifically, it is the value as measured by the method described in the Examples section.

A representative polymerization method, such as aqueous polymerization, emulsion polymerization, or reversed phase suspension polymerization, is used for polymerizing the water-soluble ethylenically unsaturated monomer. In aqueous polymerization, polymerization is performed by heating, optionally with stirring, an aqueous solution of the water-soluble ethylenically unsaturated monomer. In reversed phase suspension polymerization, polymerization is performed by heating the water-soluble ethylenically unsaturated monomer with stirring in a hydrocarbon dispersion medium.

One exemplary method for producing the water-absorbent resin particles will be hereinafter described.

Specific examples of methods for producing the water-absorbent resin particles include a method for producing the water-absorbent resin particles by performing reversed phase suspension polymerization of the water-soluble ethylenically unsaturated monomer in a hydrocarbon dispersion medium, the method including the steps of performing the polymerization in the presence of a radical polymerization initiator; and surface-crosslinking a hydrous gel obtained by the polymerization, in the presence of a surface-crosslinking agent. In the method for producing the water-absorbent resin particles of the present invention, an internal-crosslinking agent may be optionally added to the water-soluble ethylenically unsaturated monomer to obtain a hydrous gel having an internal-crosslinked structure.

### <Polymerization Step>

### [Water-Soluble Ethylenically Unsaturated Monomer]

Examples of the water-soluble ethylenically unsaturated monomer include (meth)acrylic acid ("acryl" and "methacryl" are herein collectively referred to as "(meth)acryl"; the same applies below) and salts thereof; 2-(meth)acrylamido-2-methylpropanesulfonic acid and salts thereof; nonionic monomers, such as (meth)acrylamide, N,N-dimethyl(meth)acrylamide, 2-hydroxyethyl(meth)acrylate, N-methylol(meth)acrylamide, and polyethylene glycol mono(meth)acrylate; and amino group-containing unsaturated monomers, such as N,N-diethylaminoethyl (meth)acrylate, N,N-diethylaminopropyl (meth)acrylate, and diethylaminopropyl (meth)acrylamide, as well as quaternary compounds thereof. Preferred among these water-soluble ethylenically unsaturated monomers are (meth)acrylic acid and salts thereof, (meth)acrylamide, and N,N-dimethyl(meth)acrylamide, and more preferred are (meth)acrylic acid and salts thereof, because they are readily industrially available, for example. These water-soluble ethylenically unsaturated monomers may be used alone or in combinations of two or more.

Among these water-soluble ethylenically unsaturated monomers, acrylic acid and salts thereof are widely used as raw materials of water-absorbent resin particles. Copolymers of acrylic acid and/or salts thereof with other water-soluble ethylenically unsaturated monomers as mentioned above may also be used. In this case, acrylic acid and/or a salt thereof as a main water-soluble ethylenically unsaturated monomer is preferably used in an amount of 70 to 100 mol% based on the total amount of water-soluble ethylenically unsaturated monomers.

The water-soluble ethylenically unsaturated monomer may be dispersed as an aqueous solution in a hydrocarbon dispersion medium and then subjected to reversed phase suspension polymerization. When the water-soluble ethylenically unsaturated monomer is in the form of an aqueous solution, the dispersion efficiency in the hydrocarbon dispersion medium can be increased. The concentration of the water-soluble ethylenically unsaturated monomer in the aqueous solution is preferably in the range of 20% by mass to not more than the saturation concentration. The concentration of the water-soluble ethylenically unsaturated monomer is more preferably 55% by mass or less, still more preferably 50% by mass or less, and even more preferably 45% by mass or less. On the other hand, the concentration of the water-soluble ethylenically unsaturated monomer is more preferably 25% by mass or more, still more preferably 28% by mass or more, and even more preferably 30% by mass or more.

When the water-soluble ethylenically unsaturated monomer has an acid group such as (meth)acrylic acid or 2-(meth)acrylamido-2-methylpropanesulfonic acid, the acid group may be optionally neutralized with an alkaline neutralizing agent, before the water-soluble ethylenically unsaturated monomer is used. Examples of such alkaline neutralizing agents include alkali metal salts, such as sodium hydroxide, sodium carbonate, sodium hydrogen carbonate, potassium hydroxide, and potassium carbonate; and ammonia. These alkaline neutralizing agents may be used in the form of an aqueous solution to facilitate the neutralization operation. The above-mentioned alkaline neutralizing agents may be used alone or in combinations of two or more.

The degree of neutralization of the water-soluble ethylenically unsaturated monomer with an alkaline neutralizing agent, calculated as the degree of neutralization of all acid groups in the water-soluble ethylenically unsaturated monomer, is preferably 10 to 100 mol%, more preferably 30 to 90 mol%, still more preferably 40 to 85 mol%, and even more preferably 50 to 80 mol%.

### [Radical Polymerization Initiator]

Examples of the radical polymerization initiator to be added in the polymerization step include persulfates, such as potassium persulfate, ammonium persulfate, and sodium persulfate; peroxides, such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide; and azo compounds, such as 2,2'-azobis(2-amidinopropane) dihydrochloride, 2,2'-azobis[2-(N-phenylamidino)propane] dihydrochloride, 2,2'-azobis[2-(N-allylamidino)propane] dihydrochloride, 2,2'-azobis{2-[1-(2-hydroxyethyl)-2-imidazolin-2-yl]propane} dihydrochloride, 2,2'-azobis{2-methyl-N-[1,1-bis(hydroxymethyl)-2-hydroxyethyl]propionamide}, 2,2'-azobis[2-methyl-N-(2-hydroxyethyl)-propionamide], and 4,4'-azobis(4-cyanovaleric acid). Preferred among these radical polymerization initiators are potassium persulfate, ammonium persulfate, sodium persulfate, and 2,2'-azobis(2-amidinopropane) dihydrochloride, because they are readily available and easy to handle. These radical polymerization initiators may be used alone or in combinations of two or more. The above-mentioned radical polymerization initiators may also be used in combination with a reducing agent, such as sodium sulfite, sodium hydrogensulfite, ferrous sulfate, or L-ascorbic acid, and used as a redox polymerization initiator.

The amount of the radical polymerization initiator to be used is, for example, 0.00005 to 0.01 mol per mole of the water-soluble ethylenically unsaturated monomer. When the radical polymerization initiator is used in the above-defined range of amounts, the occurrence of an abrupt polymerization reaction can be avoided, and the polymerization reaction can be completed in an appropriate period of time.

### [Internal-Crosslinking Agent]

Examples of the internal-crosslinking agent include those that can crosslink the polymer of the water-soluble ethylenically unsaturated monomer to be used, for example: unsaturated polyesters obtained by reacting polyols, such as diols and triols, e.g., (poly)ethylene glycol ["(poly)" means both cases with and without the prefix "poly"; the same applies below], (poly)propylene glycol, 1,4-butanediol, 1,6-hexanediol, trimethylolpropane, and (poly)glycerin, with unsaturated acids, such as (meth)acrylic acid, maleic acid, and fumaric acid; bisacrylamides, such as N,N-methylenebisacrylamide; di or tri(meth)acrylic acid esters obtained by reacting polyepoxides with (meth)acrylic acid; carbamyl di(meth)acrylates obtained by reacting polyisocyanates, such as tolylene diisocyanate and hexamethylene diisocyanate, with hydroxyethyl (meth)acrylate; compounds having two or more polymerizable unsaturated groups, such as allylated starch, allylated cellulose, diallyl phthalate, N,N',N"-triallyl isocyanurate, and divinylbenzene; polyglycidyl compounds, such as diglycidyl compounds, e.g., (poly)ethylene glycol diglycidyl ether, (poly)propylene glycol diglycidyl ether, and (poly)glycerin diglycidyl ether, and triglycidyl compounds; epihalohydrin compounds, such as epichlorohydrin, epibromohydrin, and **α-**methylepichlorohydrin; compounds having two or more reactive functional groups, such as isocyanate compounds, e.g., 2,4-tolylene diisocyanate and hexamethylene diisocyanate; and oxetane compounds, such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol. Among these internal-crosslinking agents, polyglycidyl compounds are preferably used, diglycidyl ether compounds are more preferably used, and (poly)ethylene glycol diglycidyl ether, (poly)propylene glycol diglycidyl ether, and (poly)glycerin diglycidyl ether are still more preferably used. These internal-crosslinking agents may be used alone or in combinations of two or more.

The amount of the internal-crosslinking agent to be used is preferably 0.000001 to 0.02 mol, more preferably 0.00001 to 0.01 mol, still more preferably 0.00001 to 0.005 mol, and even more preferably 0.00005 to 0.002 mol, per mole of the water-soluble ethylenically unsaturated monomer.

### [Hydrocarbon Dispersion Medium]

Examples of the hydrocarbon dispersion medium include C₆₋₈ aliphatic hydrocarbons, such as n-hexane, n-heptane, 2-methylhexane, 3-methylhexane, 2,3-dimethylpentane, 3-ethylpentane, and n-octane; alicyclic hydrocarbons, such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons, such as benzene, toluene, and xylene. Among these hydrocarbon dispersion media, n-hexane, n-heptane, and cyclohexane, which are readily industrially available, stable in quality, and inexpensive, are particularly suitably used. These hydrocarbon dispersion media may be used alone or in combinations of two or more. The use of a commercially available mixture of hydrocarbon dispersion media, such as Exxsol Heptane (from Exxon Mobil Corporation; containing 75 to 85% by mass of heptane and its isomeric hydrocarbons), also leads to favorable results.

The amount of the hydrocarbon dispersion medium to be used is preferably 100 to 1500 parts by mass, and more preferably 200 to 1400 parts by mass, per 100 parts by mass of a first-stage water-soluble ethylenically unsaturated monomer, in view of homogeneously dispersing the water-soluble ethylenically unsaturated monomer, and facilitating control of the polymerization temperature. As described below, reversed phase suspension polymerization is performed in one stage (single stage) or two or more multiple stages. The above-mentioned first-stage polymerization refers to the first-stage polymerization reaction in single-stage polymerization or multi-stage polymerization (the same applies below).

### [Dispersion Stabilizer]

### (Surfactant)

In reversed phase suspension polymerization, a dispersion stabilizer may be used to improve the dispersion stability of the water-soluble ethylenically unsaturated monomer in the hydrocarbon dispersion medium. A surfactant may be used as such a dispersion stabilizer.

Examples of usable surfactants include sucrose fatty acid esters, polyglycerol fatty acid esters, sorbitan fatty acid esters, polyoxyethylene sorbitan fatty acid esters, polyoxyethylene glycerin fatty acid esters, sorbitol fatty acid esters, polyoxyethylene sorbitol fatty acid esters, polyoxyethylene alkyl ethers, polyoxyethylene alkyl phenyl ethers, polyoxyethylene castor oil, polyoxyethylene hydrogenated castor oil, alkyl allyl formaldehyde condensate polyoxyethylene ethers, polyoxyethylene-polyoxypropylene block copolymers, polyoxyethylene polyoxypropyl alkyl ethers, polyethylene glycol fatty acid esters, alkyl glucosides, N-alkyl glyconamides, polyoxyethylene fatty acid amides, polyoxyethylene alkylamines, polyoxyethylene alkyl ether phosphates, and polyoxyethylene alkyl allyl ether phosphates. Among these surfactants, sorbitan fatty acid esters, polyglycerol fatty acid esters, and sucrose fatty acid esters are particularly preferably used, in view of dispersion stability of the monomer. These surfactants may be used alone or in combinations of two or more.

The amount of the surfactant to be used is preferably 0.1 to 30 parts by mass, and more preferably 0.3 to 20 parts by mass, per 100 parts by mass of the first-stage water-soluble ethylenically unsaturated monomer.

### (Polymeric Dispersion Agent)

A polymeric dispersion agent may be used in combination with the above-described surfactant, as the dispersion stabilizer to be used in reversed phase suspension polymerization.

Examples of the polymeric dispersion agent include maleic anhydride modified polyethylene, maleic anhydride modified polypropylene, maleic anhydride modified ethylene-propylene copolymers, maleic anhydride modified EPDM (ethylene-propylene-diene terpolymers), maleic anhydride modified polybutadiene, maleic anhydride-ethylene copolymers, maleic anhydride-propylene copolymers, maleic anhydride-ethylene-propylene copolymers, maleic anhydride-butadiene copolymers, polyethylene, polypropylene, ethylene-propylene copolymers, oxidized polyethylene, oxidized polypropylene, oxidized ethylene-propylene copolymers, ethylene-acrylic acid copolymers, ethyl cellulose, and ethyl hydroxyethyl cellulose. Among these polymeric dispersion agents, maleic anhydride modified polyethylene, maleic anhydride modified polypropylene, maleic anhydride modified ethylene-propylene copolymers, maleic anhydride-ethylene copolymers, maleic anhydride-propylene copolymers, maleic anhydride-ethylene-propylene copolymers, polyethylene, polypropylene, ethylene-propylene copolymers, oxidized polyethylene, oxidized polypropylene, and oxidized ethylene-propylene copolymers are particularly preferably used, in view of dispersion stability of the monomer. These polymeric dispersion agents may be used alone or in combinations of two or more.

The amount of the polymeric dispersion agent to be used is preferably 0.1 to 30 parts by mass, and more preferably 0.3 to 20 parts by mass, per 100 parts by mass of the first-stage water-soluble ethylenically unsaturated monomer.

### [Other Components]

In the method for producing the water-absorbent resin particles, other components may be optionally added to the aqueous solution containing the water-soluble ethylenically unsaturated monomer, which is then subjected to reversed phase suspension polymerization. Various additives such as thickeners and chain transfer agents can be added as the other components.

By way of example, a thickener may be added to the aqueous solution containing the water-soluble ethylenically unsaturated monomer, which is then subjected to reversed phase suspension polymerization. By thus adding a thickener to adjust the viscosity of the aqueous solution, the median particle size obtained by reversed phase suspension polymerization can be controlled.

Examples of usable thickeners include hydroxyethylcellulose, hydroxypropylcellulose, methylcellulose, carboxymethylcellulose, polyacrylic acid, (partially) neutralized polyacrylic acid, polyethylene glycol, polyacrylamide, polyethyleneimine, dextrin, sodium alginate, polyvinyl alcohol, polyvinylpyrrolidone, and polyethylene oxide. Assuming that the stirring rate during the polymerization is the same, the higher the viscosity of the aqueous solution containing the water-soluble ethylenically unsaturated monomer, the larger the primary particles and/or secondary particles of the resulting particles tend to be.

### [Reversed Phase Suspension Polymerization]

To perform reversed phase suspension polymerization, for example, an aqueous monomer solution containing the water-soluble ethylenically unsaturated monomer is dispersed in a hydrocarbon dispersion medium, in the presence of a dispersion stabilizer. Here, as long as the dispersion stabilizer (a surfactant and a polymeric dispersion agent) is added before the beginning of the polymerization reaction, it may be added either before or after the addition of the aqueous monomer solution.

In particular, in view of readily reducing the amount of the hydrocarbon dispersion medium remaining in the resulting water-absorbent resin particles, it is preferred to disperse the aqueous monomer solution in the hydrocarbon dispersion medium in which a polymeric dispersion agent is dispersed and then disperse a surfactant therein, followed by polymerization.

Reversed phase suspension polymerization as described above can be performed in one stage or two or more multiple stages. In view of improving productivity, reversed phase suspension polymerization is preferably performed in two or three stages.

Reversed phase suspension polymerization in two or more multiple stages may be performed as follows: the first stage of reversed phase suspension polymerization is performed; subsequently, the water-soluble ethylenically unsaturated monomer is added to and mixed with the reaction mixture obtained by the first-stage polymerization reaction, and the second and subsequent stages of reversed phase suspension polymerization are performed in the same manner as in the first stage. In each of the second and subsequent stages of reversed phase suspension polymerization, it is preferred to add, in addition to the water-soluble ethylenically unsaturated monomer, a radical polymerization initiator within the above-described range of molar ratios of each component relative to the water-soluble ethylenically unsaturated monomer, based on the amount of the water-soluble ethylenically unsaturated monomer added in each of the second and subsequent stages of reversed phase suspension polymerization, and then perform reversed phase suspension polymerization. In the second and subsequent stages of polymerization, an internal-crosslinking agent may also be optionally added to the water-soluble ethylenically unsaturated monomer.

The reaction temperature during the polymerization reaction is preferably 20 to 110°C, and more preferably 40 to 90°C, in view of allowing the polymerization to proceed quickly to reduce the polymerization time for improved economic efficiency, and readily removing the heat of polymerization to perform the reaction smoothly.

### <Surface-Crosslinking Step>

Next, the water-absorbent resin particles of the present invention are obtained by crosslinking the hydrous gel having an internal-crosslinked structure obtained by polymerization of the water-soluble ethylenically unsaturated monomer, by adding a surface-crosslinking agent thereto (surface-crosslinking reaction). The surface-crosslinking reaction is preferably performed in the presence of a surface-crosslinking agent, after the polymerization of the water-soluble ethylenically unsaturated monomer. By thus subjecting the hydrous gel having an internal-crosslinked structure to the surface-crosslinking reaction after the polymerization, it is possible to obtain water-absorbent resin particles having an increased crosslinking density near the surfaces of the water-absorbent resin particles to exhibit improvement in various kinds of performance, such as water-absorption capacity under load.

Examples of the surface-crosslinking agent include compounds with two or more reactive functional groups. Examples include polyols, such as ethylene glycol, propylene glycol, 1,4-butanediol, diethylene glycol, triethylene glycol, trimethylolpropane, glycerin, polyoxyethylene glycol, polyoxypropylene glycol, and polyglycerin; polyglycidyl compounds, such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; haloepoxy compounds, such as epichlorohydrin, epibromohydrin, and α-methyl epichlorohydrin; isocyanate compounds, such as 2,4-tolylene diisocyanate and hexamethylene diisocyanate; oxetane compounds, such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol; oxazoline compounds, such as 1,2-ethylenebisoxazoline; carbonate compounds (e.g., alkylene carbonates), such as ethylene carbonate, propylene carbonate, 4,5-dimethyl-1,3-dioxolan-2-one, 4,4-dimethyl-1,3-dioxolan-2-one, 4-ethyl-1,3-dioxolan-2-one, 4-hydroxymethyl-1,3-dioxolan-2-one, 1,3-dioxan-2-one, 4-methyl-1,3-dioxan-2-one, 4,6-dimethyl-1,3-dioxan-2-one, and 1,3-dioxopan-2-one; and hydroxyalkylamide compounds, such as bis[N,N-di(β-hydroxyethyl)]adipamide. Preferred among these surface-crosslinking agents are polyglycidyl compounds, such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether. These surface-crosslinking agents may be used alone or in combinations of two or more.

The amount of the surface-crosslinking agent to be used is preferably 0.00001 to 0.01 mol, more preferably 0.00005 to 0.005 mol, and still more preferably 0.0001 to 0.002 mol, per mole of the total amount of the water-soluble ethylenically unsaturated monomer used for polymerization.

The surface-crosslinking agent may be added as is or as an aqueous solution. Optionally, a solution of the surface-crosslinking agent in a hydrophilic organic solvent may be added. Examples of the hydrophilic organic solvent include lower alcohols, such as methyl alcohol, ethyl alcohol, n-propyl alcohol, and isopropyl alcohol; ketones, such as acetone and methyl ethyl ketone; ethers, such as diethyl ether, dioxane, and tetrahydrofuran; amides, such as N,N-dimethylformamide; and sulfoxides, such as dimethylsulfoxide. These hydrophilic organic solvents may be used alone, in combinations of two or more, or as a solvent mixture with water.

The surface-crosslinking agent may be added at any time after the polymerization reaction of the water-soluble ethylenically unsaturated monomer is substantially completed. The surface-crosslinking agent is preferably added in the presence of 1 to 400 parts by mass of water, more preferably 5 to 200 parts by mass of water, still more preferably 10 to 100 parts by mass of water, even more preferably 20 to 60 parts by mass of water, per 100 parts by mass of the water-soluble ethylenically unsaturated monomer. The amount of water herein refers to the total amount of the water contained in the reaction system and the water optionally used during the addition of the surface-crosslinking agent.

The reaction temperature during the surface-crosslinking reaction is preferably 50 to 250°C, more preferably 60 to 180°C, still more preferably 60 to 140°C, and even more preferably 70 to 120°C. The reaction time of the surface-crosslinking reaction is preferably 1 to 300 minutes, and more preferably 5 to 200 minutes.

### <Drying Step>

After reversed phase suspension polymerization is performed as described above, the method may include a drying step of adding external energy, such as heat, to remove the water, the hydrocarbon dispersion medium, and the like by distillation. To remove the water in the hydrous gel after reversed phase suspension polymerization, the system in which the hydrous gel is dispersed in the hydrocarbon dispersion medium is heated to distill the water and the hydrocarbon dispersion medium out of the system by azeotropic distillation. Here, if the distilled hydrocarbon dispersion medium only is returned into the system, continuous azeotropic distillation can be performed. This is preferable in view of preventing deterioration of the resin, because the temperature within the system during drying is maintained at a temperature not higher than the azeotropic temperature with the hydrocarbon dispersion medium. Subsequently, the water and the hydrocarbon dispersion medium are distilled off to obtain water-absorbent resin particles. By controlling the treatment conditions for the drying step after the polymerization to adjust the amount of water to be removed, various kinds of performance of the resulting water-absorbent resin particles can be controlled.

In the drying step, the drying treatment by distillation may be performed under atmospheric pressure or reduced pressure. The drying treatment may also be performed in a stream of nitrogen or the like, in view of improving the drying efficiency. When the drying treatment is performed under atmospheric pressure, the drying temperature is preferably 70 to 250°C, more preferably 80 to 180°C, still more preferably 80 to 140°C, and even more preferably 90 to 130°C. When the drying treatment is performed under reduced pressure, the drying temperature is preferably 40 to 160°C, and more preferably 50 to 110°C.

When the surface-crosslinking step with a surface-crosslinking agent is performed after the polymerization of the monomer by reversed phase suspension polymerization, the drying step by distillation is performed as described above, after the completion of the surface-crosslinking step. Alternatively, the surface-crosslinking step and the drying step may be performed simultaneously.

The water-absorbent resin composition of the present invention may contain additives suitable for its purpose. Examples of such additives include inorganic powders, surfactants, oxidizing agents, reducing agents, metal chelating agents, radical chain inhibitors, antioxidants, and anti-bacterial agents. For example, when 0.05 to 5 parts by mass of amorphous silica as an inorganic powder is added to 100 parts by mass of the water-absorbent resin particles, the flowability of the water-absorbent resin composition can be further improved. The additives are preferably hydrophilic or water-soluble.

In the water-absorbent resin composition of the present invention, the water-absorbent resin particles content (excluding the additives) is preferably 70% by mass or more, more preferably 80% by mass or more, and still more preferably 90% by mass or more.

The water-absorbent resin composition of the present invention can be produced by, for example, mixing the water-absorbent resin particles and the activated carbon in a solid state.

### 2. Absorbent Material and Absorbent Article

The water-absorbent resin composition of the present invention constitutes an absorbent material used for hygienic materials, such as sanitary items and disposable diapers, for example, and is suitably used for an absorbent article including the absorbent material.

Herein, the absorbent material obtained using the water-absorbent resin composition of the present invention contains the particulate water-absorbent resin composition of the present invention. The absorbent material may further contain hydrophilic fibers. Examples of the structure of the absorbent material include a sheet-like structure in which the water-absorbent resin particles are fixed on a nonwoven fabric or between a plurality of nonwoven fabrics; a mixed dispersion obtained by mixing the particulate water-absorbent resin composition and hydrophilic fibers to give a homogeneous composition; a sandwich structure in which the particulate water-absorbent resin composition is sandwiched between layered hydrophilic fibers; and a structure in which the particulate water-absorbent resin composition and hydrophilic fibers are wrapped in a tissue. The absorbent material may also contain other components, for example, adhesive binders such as thermal bonding synthetic fibers, hot melt adhesives, and adhesive emulsions, for improving the shape retention properties of the absorbent material.

The water-absorbent resin composition content in the absorbent material is preferably 5 to 100% by mass, more preferably 10 to 95% by mass, still more preferably 20 to 90% by mass, and even more preferably 30 to 80% by mass.

Examples of the hydrophilic fibers include cellulose fibers, such as cotton-like pulp made from wood, mechanical pulp, chemical pulp, and semi-chemical pulp; artificial cellulose fibers, such as rayon and acetate; and hydrophilized fibers made of synthetic resins, such as polyamides, polyesters, and polyolefins. The hydrophilic fibers typically have an average fiber length of 0.1 to 10 mm. Alternatively, the average fiber length may be 0.5 to 5 mm.

It is possible to obtain the absorbent article of the present invention by holding the absorbent material obtained using the particulate water-absorbent resin composition of the present invention between a liquid-permeable sheet (top sheet) that allows a liquid to pass through and a liquid-impermeable sheet (back sheet) that does not allow a liquid to pass through. The liquid-permeable sheet is positioned on the side that is brought into contact with the body, and the liquid-impermeable sheet is positioned opposite to the side that is brought into contact with the body.

Examples of the liquid-permeable sheet include porous synthetic resin sheets and nonwoven fabrics of the types such as air-through type, spunbond type, chemical bond type, and needle punch type, which are made of fibers of polyethylene, polypropylene, polyester, and the like. Examples of the liquid-impermeable sheet include synthetic resin films made of resins such as polyethylene, polypropylene, and polyvinyl chloride.

### Examples

The present invention will be hereinafter described in detail with reference to examples and comparative examples. However, the present invention is not limited to the examples.

The water-absorbent resin particles, the activated carbons, and the water-absorbent resin compositions obtained in the examples and comparative examples were evaluated using the tests as described below. Unless otherwise indicated, the measurement was performed in an environment at a temperature of 25 ± 2°C and a humidity of 50 ± 10%.

### [Evaluation of the Water-Absorbent Resin Particles]

### <Physiological Saline Retention Amount>

2.0 g of the water-absorbent resin particles were weighed into a cotton bag (Cottonbroad No. 60, 100 mm in width × 200 mm in length), and the cotton bag was placed in a 500 mL beaker. 500 g of a 0.9% by mass aqueous solution of sodium chloride (physiological saline) was poured at once into the cotton bag containing the water-absorbent resin particles so as to prevent formation of unswollen lumps, then the top of the cotton bag was closed with a rubber band, and the cotton bag was allowed to stand for 30 minutes to cause the water-absorbent resin particles to swell. After 30 minutes, the cotton bag was dehydrated for 1 minute using a dehydrator (product number: H-122 from Kokusan Co., Ltd.) set at a centrifugal force of 167 G, and a mass Wd (g) of the cotton bag containing the swollen gel after dehydration was measured. The same operation was performed without adding the water-absorbent resin particles, and an empty mass We (g) of the cotton bag upon wetting was measured. The physiological saline retention amount was calculated according to the following equation:
Physiological saline retention amount (g/g) = [Wd - We]/2.0

### <Measurement of Water-Absorption Rate by the Vortex Method>

50 ± 0.1 g of physiological saline adjusted to a temperature of 25 ± 0.2°C in a thermostat was weighed into a 100 mL beaker and stirred with a magnetic stirrer bar (8 mm (p × 30 mm, without a ring) to form a vortex at a rotation speed of 600 rpm. 2.0 ± 0.002 g of the water-absorbent resin particles were added at once into the physiological saline, and the time (seconds) from the addition of the water-absorbent resin particles to the point at which the vortex on the liquid surface disappeared was measured. The measured time was determined as the water-absorption rate of the water-absorbent resin particles. This water-absorption rate is also referred to as the Vortex method or vortex time.

### <Median Particle Size (Particle Size Distribution)>

50 g of the water-absorbent resin particles were used for median particle size (particle size distribution) measurement. JIS standard sieves having mesh sizes of 850 µm, 500 µm, 425 µm, 300 µm, 250 µm, 180 µm, and 150 µm, and a receiving tray were combined in that order from the top. The water-absorbent resin particles were placed on the top sieve of the combined sieves, and classified by shaking for 20 minutes using a Ro-Tap shaker. After the classification, the particle size distribution was determined by calculating the mass of the water-absorbent resin particles remaining on each sieve as the mass percentage relative to the total mass. With regard to this particle size distribution, the mass percentage of the water-absorbent resin particles remaining on each sieve was integrated in descending order of particle size. In this manner, the relationship between the sieve mesh size and the cumulative value of the mass percentage of the water-absorbent resin particles remaining on each sieve was plotted on logarithmic probability paper. The plots on the probability paper were connected with straight lines, and a particle size equivalent to a 50% by mass cumulative mass percentage was determined as the median particle size.

### <Physiological Saline Absorption Amount Under a Load of 4.14 kPa>

Physiological saline absorption amount under a load of 4.14 kPa (water absorption amount under load) was measured using the measurement apparatus as schematically shown in Fig. 1. Two measurements were made for one type of water-absorbent resin particles, and the average value was obtained. The measurement apparatus includes a burette unit 1, a clamp 3, a conduit 5, a stand 11, a measurement table 13, and a measurement unit 4 placed on the measurement table 13. The burette unit 1 has a graduated burette tube 21, a rubber stopper 23 for sealing the upper opening of the burette tube 21, a cock 22 connected to the lower end of the burette tube 21, an air inlet tube 25 connected to a lower portion of the burette tube 21, and a cock 24. The burette unit 1 is fixed by the clamp 3. The flat measurement table 13 has a through hole 13a with a diameter of 2 mm formed in the central portion thereof, and is supported by the variable-height stand 11. The through hole 13a of the measurement table 13 and the cock 22 of the burette unit 1 are connected through the conduit 5. The conduit 5 has an inner diameter of 6 mm.

The measurement unit 4 includes a cylinder 31 made of plexiglas, a polyamide mesh 32 bonded to one opening of the cylinder 31, and a weight 33 vertically movable within the cylinder 31. The cylinder 31 is placed on the measurement table 13 with the polyamide mesh 32 therebetween. The cylinder 31 has an inner diameter of 20 mm. The polyamide mesh 32 has a mesh size of 75 µm (200 mesh). The weight 33 has a diameter of 19 mm and a mass of 119.6 g and, as described later, can apply a load of 4.14 kPa (0.6 psi) to water-absorbent resin particles 10a uniformly disposed on the polyamide mesh 32.

First, the cock 22 and the cock 24 of the burette unit 1 were closed, and 0.9% by mass physiological saline adjusted to 25°C was placed in the burette tube 21 through the upper opening of the burette tube 21. Subsequently, the upper opening of the burette tube 21 was sealed with the rubber stopper 23, and then the cock 22 and the cock 24 were opened. The conduit 5 was filled with the 0.9% by mass physiological saline 50 to prevent air bubbles from entering. The height of the measurement table 13 was adjusted so that the height of the surface of the 0.9% by mass physiological saline reached into the through hole 13a was equal to the height of the upper surface of the measurement table 13. After the adjustment, the height of the surface of the 0.9% by mass physiological saline 50 in the burette tube 21 was read on the graduations of the burette tube 21, and this position was determined as a zero point (reading at 0 seconds).

In the measurement unit 4, 0.10 g of the water-absorbent resin particles 10a were uniformly disposed on the polyamide mesh 32 in the cylinder 31, the weight 33 was disposed on the water-absorbent resin particles 10a, and the cylinder 31 was installed such that the central portion thereof aligned with the conduit opening at the central portion of the measurement table 13. An amount of reduction Wc (mL) in the physiological saline in the burette tube 21 at 60 minutes after the beginning of absorption of the physiological saline by the water-absorbent resin particles 10a through the conduit 5 (that is, the amount of the physiological saline absorbed by the water-absorbent resin particles 10a) was read, and the physiological saline absorption capacity under a load of 4.14 kPa of the water-absorbent resin particles 10a was calculated using the following equation:
Physiological saline absorption capacity (mL/g) under a load of 4.14 kPa = Wc (mL)/mass (g) of the water-absorbent resin particles

### <BET Specific Surface Area of the Water-Absorbent Resin Particles>

The water-absorbent resin particles to be measured were adjusted to a particle size such that they passed through a sieve with a mesh size of 400 µm and retained on a sieve with a mesh size of 300 µm, and then used for specific surface area measurement. Subsequently, 10 g of the classified sample was dispersed in 100 g of ethanol and washed for 5 minutes in an ultrasonic cleaner (US-103 from SND Co., Ltd.) and then filtered through a sieve with a mesh size of 300 µm. Two more washing operations were repeated in the same manner to give a measurement sample washed a total of three times. This sample was dried under degassing conditions of evacuation with heating at 100°C for 16 hours. Then, an adsorption isotherm was measured at a temperature of 77 K using a specific surface area analyzer (AUTOSORB-1 from Quantachrome), by the method using krypton gas as the adsorption gas, and the specific surface area was determined from a multi-point BET plot as the BET specific surface area of the water-absorbent resin particles.

### [Preparation of Activated Carbons]

### <Activated Carbon A>

An activated carbon (product name: FP-3 from Osaka Gas Chemicals Co., Ltd.) was prepared having a BET specific surface area of 1619 m²/g, a median particle size of 38 µm, a residue on ignition of 0.1%, a loss on drying of 0.8%, an iodine adsorption amount of 1560 mg/g, a pH of 7.1, and a crushed shape. This activated carbon was designated as activated carbon A.

### <Activated Carbon B>

An activated carbon (product name: FPG-1 from Osaka Gas Chemicals Co., Ltd.) was prepared having a BET specific surface area of 1495 m²/g, a median particle size of 7 µm, a residue on ignition of 3.2%, a loss on drying of 3.4%, an iodine adsorption amount of 1600 mg/g, a pH of 10.2, and a crushed shape. This activated carbon was designated as activated carbon B.

### [Evaluation of Activated Carbon]

### <Median Particle Size (Laser Diffraction) of Activated Carbon>

The median particle size (D50 (median size), volume-based) of the activated carbon used was measured using a laser diffraction particle size distribution analyzer (SALD-2300 from Shimadzu Corporation).

### <BET Specific Surface Area of Activated Carbon>

0.1 g of the activated carbon to be measured was dried under degassing conditions of evacuation with heating at 60°C for 24 hours, using a pretreatment apparatus (BELPREP VAC II from MicrotracBel Corporation). Then, an adsorption isotherm was measured at a temperature of 77 K using a specific surface area analyzer (BELSORP MINI II from MicrotracBel Corporation), by the method using nitrogen gas as the adsorption gas, and the specific surface area was determined from a multi-point BET plot as the BET specific surface area of the activated carbon.

### [Production of Water-Absorbent Resin Compositions]

### <Example 1>

A 110 mm inner diameter, 2 L volume, cylindrical round-bottomed separable flask with four side wall baffles (baffle width: 7 mm) was prepared which was equipped with a reflux condenser, a dropping funnel, a nitrogen gas inlet tube, and a stirrer. The stirrer was mounted with stirring blades having two stages of four inclined paddle blades surface-treated with a fluororesin and having a blade diameter of 50 mm. In the prepared separable flask, 451.4 g of n-heptane and 1.288 g of sorbitan monolaurate (trade name: NONION LP-20R, HLB value: 8.6, from NOF CORPORATION) were mixed. The mixture in the separable flask was heated to 50°C while stirring with the stirrer to dissolve the sorbitan monolaurate in the n-heptane. The resulting solution was cooled to 40°C.

92.0 g of an 80.5% by mass aqueous solution of acrylic acid (acrylic acid: 1.03 mol) was placed in a 500 mL inner volume Erlenmeyer flask. 147.7 g of a 20.9% by mass aqueous solution of sodium hydroxide was added dropwise into the aqueous solution of acrylic acid in the flask, with external ice-cooling, to partially neutralize the acrylic acid. Subsequently, 0.1011 g (0.374 mmol) of potassium persulfate was added as a water-soluble radical polymerization initiator and dissolved in the aqueous solution to prepare an aqueous monomer solution.

The resulting aqueous monomer solution was added to the separable flask containing the solution containing the sorbitan monolaurate, and the system was sufficiently purged with nitrogen. While stirring with the stirrer at a rotation speed of 700 rpm, the reaction mixture in the separable flask was maintained in a warm water bath at 70°C for 60 minutes, causing the polymerization reaction to proceed.

To the reaction mixture containing the polymer as a hydrous gel produced by the polymerization reaction, a dispersion of 0.092 g of amorphous silica (Tokusil NP-S from Oriental Silicas Corporation) in 100 g of n-heptane was added, and the reaction mixture was stirred for 10 minutes. The separable flask was immersed in an oil bath at 125°C to remove 98.5 g of water out of the system by azeotropic distillation. Then, 4.14 g of a 2% by mass aqueous solution of ethylene glycol diglycidyl ether (ethylene glycol diglycidyl ether: 0.475 mmol) was added as a surface-crosslinking agent, and the flask was maintained at an internal temperature of 80 ± 2°C for 2 hours to cause the surface-crosslinking reaction to proceed.

The reaction mixture was heated to 125°C to evaporate the water and n-heptane, thus giving a dried product of the polymer particles. This dried product was passed through a sieve with a mesh size of 850 µm to give 86.3 g of water-absorbent resin particles. The water-absorbent resin particles had a physiological saline retention amount of 35 g/g, a water-absorption rate of 4 seconds, a median particle size of 360 µm, a physiological saline absorption amount under a load of 4.14 kPa of 11 mL/g, and a BET specific surface area of 0.150 m²/g. 0.3 part by mass of activated carbon A was added to 100 parts by mass of the obtained water-absorbent resin particles, and the mixture was mixed for 30 minutes using a cross-rotary mixer from Meiwa Kogyo Co., Ltd. (conditions: revolution speed 50 rpm, rotation speed 50 rpm) to give a water-absorbent resin composition.

### <Example 2>

A 11 cm inner diameter, 2 L volume, cylindrical round-bottomed separable flask was prepared which was equipped with a reflux condenser, a dropping funnel, a nitrogen gas inlet tube, and a stirrer with stirring blades having two stages of four inclined paddle blades with a blade diameter of 5 cm. As a hydrocarbon dispersion medium, 293 g of n-heptane was placed in this flask, and 0.736 g of a maleic anhydride-modified ethylene-propylene copolymer (Hi-Wax 1105A from Mitsui Chemicals, Inc.) was added as a polymeric dispersion agent. The mixture was heated with stirring to 80°C to dissolve the dispersion agent and then cooled to 50°C. Separately, in a 300 mL inner volume beaker, 92.0 g (1.03 mol) of an 80.5% by mass aqueous solution of acrylic acid was placed as a water-soluble ethylenically unsaturated monomer, and 147.7 g of a 20.9% by mass aqueous solution of sodium hydroxide was added dropwise, while cooling with ice water, to achieve 75 mol% neutralization. Then, 0.092 g of hydroxyethyl cellulose (HEC AW-15F from Sumitomo Seika Chemicals Co., Ltd.) as a thickener, 0.0736 g (0.272 mmol) of potassium persulfate as a water-soluble radical polymerization agent, and 0.010 g (0.057 mmol) of ethylene glycol diglycidyl ether as an internal-crosslinking agent were added and dissolved to prepare a first-stage aqueous solution. Then, the aqueous solution prepared as above was added to the separable flask and stirred for 10 minutes. Then, a surfactant solution prepared in a 20 mL vial by heating and dissolving 0.736 g of sucrose stearate having an HLB of 3 (Ryoto sugar ester S-370 from Mitsubishi-Kagaku Foods Corporation) as a surfactant in 6.62 g of n-heptane was further added. The system was sufficiently purged with nitrogen while stirring with the stirrer at a rotation speed of 550 rpm. Then, the flask was immersed in a water bath at 70°C and heated, and polymerization was performed for 60 minutes to give a first-stage polymerization slurry.

Separately, in another 500 mL inner volume beaker, 128.8 g (1.43 mol) of an 80.5% by mass aqueous solution of acrylic acid was placed as a water-soluble ethylenically unsaturated monomer, and 159.0 g of a 27% by mass aqueous solution of sodium hydroxide was added dropwise, while cooling with ice water, to achieve 75 mol% neutralization. Then, 0.103 g (0.381 mmol) of potassium persulfate as a water-soluble radical polymerization initiator and 0.0117 g (0.067 mmol) of ethylene glycol diglycidyl ether as an internal-crosslinking agent were added and dissolved to prepare a second-stage aqueous solution.

The separable flask system was cooled to 27°C while stirring with the stirrer at a rotation speed of 1000 rpm. Then, the entire amount of the second-stage aqueous solution was added to the first-stage polymerization slurry, and the system was purged with nitrogen for 30 minutes. Then, the flask was again immersed in a water bath at 70°C and heated, and the polymerization reaction was performed for 60 minutes to give a hydrous gel polymer.

Then, the flask was immersed in an oil bath set at 125°C to remove 256.1 g of water out of the system, while refluxing the n-heptane, by azeotropic distillation of the water and n-heptane. Then, 4.42 g (0.507 mmol) of a 2% by mass aqueous solution of ethylene glycol diglycidyl ether was added to the flask as a surface-crosslinking agent, and the flask was maintained at 83°C for 2 hours.

Then, the n-heptane was evaporated at 125°C to dry the reaction mixture, and the resulting product was subsequently passed through a sieve with a mesh size of 850 µm to give 230.0g of water-absorbent resin particles. The water-absorbent resin particles had a physiological saline retention amount of 41 g/g, a water-absorption rate of 29 seconds, a median particle size of 312 µm, a physiological saline absorption amount under a load of 4.14 kPa of 19 mL/g, and a BET specific surface area of 0.037 m²/g. 0.3 part by mass of activated carbon A was added to 100 parts by mass of the obtained water-absorbent resin particles, and the mixture was mixed for 30 minutes using a cross-rotary mixer from Meiwa Kogyo Co., Ltd. (conditions: revolution speed 50 rpm, rotation speed 50 rpm) to give a water-absorbent resin composition.

### <Example 3>

The procedure of Example 2 was repeated, except that activated carbon A was replaced by activated carbon B, and 0.3 part by mass of activated carbon B was added to 100 parts by mass of the water-absorbent resin particles, and the mixture was mixed for 30 minutes using a cross-rotary mixer from Meiwa Kogyo Co., Ltd. (conditions: revolution speed 50 rpm, rotation speed 50 rpm), to give a water-absorbent resin composition.

### <Example 4>

The procedure of Example 2 was repeated, except that, before the step of adding activated carbon A, 0.5 part by mass of amorphous silica (Tokusil NP-S from Oriental Silicas Corporation) was added to 100 parts by mass of the obtained water-absorbent resin particles, and the mixture was mixed for 30 minutes using a cross-rotary mixer from Meiwa Kogyo Co., Ltd. (conditions: revolution speed 50 rpm, rotation speed 50 rpm), and then activated carbon A was further added to the mixture such that the amount of activated carbon A was 0.3 part by mass per 100 parts by mass of the water-absorbent resin particles, and the mixture was mixed for 30 minutes using a cross-rotary mixer from Meiwa Kogyo Co., Ltd. (conditions: revolution speed 50 rpm, rotation speed 50 rpm), to give a water-absorbent resin composition.

### <Comparative Example 1>

The procedure of Comparative Example 1 was repeated, except that when producing a hydrous gel polymer, the separable flask system was cooled to 25°C, and then the entire amount of the second-stage aqueous solution was added to the first-stage polymerization slurry, and that the amount of water to be removed by azeotropic distillation of the n-heptane and water was changed to 254.5 g, to give 219.9 g of water-absorbent resin particles. The water-absorbent resin particles had a physiological saline retention amount of 40 g/g, a median particle size of 386 µm, a water-absorption rate of 42 seconds, a physiological saline absorption amount under a load of 4.14 kPa of 21 mL/g, and a BET specific surface area of 0.032 m²/g. 0.3 part by mass of activated carbon A was added to 100 parts by mass of the obtained water-absorbent resin particles, and the mixture was mixed for 30 minutes using a cross-rotary mixer from Meiwa Kogyo Co., Ltd. (conditions: revolution speed 50 rpm, rotation speed 50 rpm) to give a water-absorbent resin composition.

### <Comparative Example 2>

The procedure of Example 2 was repeated, except that when producing a hydrous gel polymer, the separable flask system was cooled to 31°C, and then the entire amount of the second-stage aqueous solution was added to the first-stage polymerization slurry, to give 232.0 g of water-absorbent resin particles. The water-absorbent resin particles had a physiological saline retention amount of 42 g/g, a water-absorption rate of 7 seconds, and a median particle size of 66 µm. 2.0 part by mass of amorphous silica (Tokusil NP-S from Oriental Silicas Corporation) was added to 100 parts by mass of the obtained water-absorbent resin particles, and the mixture was mixed for 30 minutes using a cross-rotary mixer from Meiwa Kogyo Co., Ltd. (conditions: revolution speed 50 rpm, rotation speed 50 rpm), and then activated carbon A was further added to the mixture such that the amount of activated carbon A was 0.3 part by mass per 100 parts by mass of the water-absorbent resin particles, and the mixture was mixed for 30 minutes using a cross-rotary mixer from Meiwa Kogyo Co., Ltd. (conditions: revolution speed 50 rpm, rotation speed 50 rpm) to give a water-absorbent resin composition.

### <Comparative Example 3>

The water-absorbent resin particles obtained in Example 2 alone were used as Comparative Example 3.

### [Evaluation of the Water-Absorbent Resin Composition]

### <Heat Release Test>

The following measurement was performed in an environment at a temperature of 25 ± 2°C and a humidity of 50 ± 10%. A 300 mL volume (inner diameter: 65 mm, height: 120 mm) stainless steel Dewar bottle (D-301 from Thermos) with a cork stopper was prepared. A drip-proof digital thermometer (CT-422WR from CUSTOM Corporation) had been inserted at a position 18 mm in the circumference from the center of the cork stopper, with its measurement tip being fixed at a position 5 mm above the bottom of the stainless steel Dewar bottle. A magnetic stirrer bar (30 mm × 8 mm ϕ) and 50.00 g of physiological saline at 24.9°C were placed in the stainless steel Dewar bottle, and the physiological saline was stirred with the magnetic stirrer at 600 rpm. While stirring the physiological saline, 10.0000 ± 0.0002 g of the water-absorbent resin composition was added into the stainless steel Dewar bottle, which was then covered with the cork stopper with the thermometer. A time t₁ (seconds) required for the temperature displayed on the thermometer to increase by 2.0°C and reach 26.9°C after the addition of water-absorbent resin composition and a temperature Ti (°C) displayed 5 minutes after the addition of the water-absorbent resin composition were measured. The values calculated from the measured values using the following equations were defined as the heat release rate and the heat release temperature:
Heat release rate (°C/sec) = 2.0 (°C)/t₁ (sec)
Heat release temperature (°C) = T₁ (°C) - 24.9 (°C)

### <Deodorization Test>

The deodorizing performance of each water-absorbent resin composition was evaluated using the following procedure. First, 3.75 g of the water-absorbent resin composition was uniformly spread on 2.5 g of 12 cm × 11 cm ground pulp, and 2.5 g of 12 cm × 11 cm ground pulp was overlaid thereon to prepare a sheet-like absorbent material, and then the absorbent material was pressed by applying a load of 284 kPa to the entire absorbent material for 30 seconds. A stainless steel petri dish (ϕ 120 mm × 25 mm) was prepared, and the pressed absorbent material was placed therein. 150 mL of test human urine was added thereto (the test human urine was prepared by collecting urine from seven adults aged 40 to 60 and mixing, and used within the day). The stainless steel petri dish containing the absorbent material was placed in a 10 L polyester sampling bag (PAAAK10 from GL Sciences Inc.), and the bag was sealed by pressure-bonding with a heat sealer (model number: FI-450-5 from Fuji Impulse Co., Ltd). A 1000 mL volume syringe was used to remove the gas phase inside the sampling bag and inject 5 L of dry air instead. Then, the sampling bag was placed and stored in a constant temperature and humidity chamber (LHU-113 from Espec Corporation) set at a temperature of 36 ± 1°C and a humidity of 60 ± 5%. After 1 hour, the sampling bag was removed from the constant temperature and humidity chamber, and 1000 mL of the gas phase was collected into a previously degassed 1 L sampling bag (PAAAK1 from GL Sciences Inc.). The same procedure was also performed for the water-absorbent resin particles not blended with activated carbon (Comparative Example 3). The gas phase was collected, and the odor of this gas phase was used as a control. Six panelists were asked to smell the odor collected from each of the water-absorbent resin compositions of Examples 1 to 4, the water-absorbent resin compositions of Comparative Examples 1 and 2, and the control of Comparative Example 3, and evaluate according to the following evaluation criteria. The average value is shown in Table 1.
5: Increased odor compared to the control
4: No change in odor compared to the control
3: Slightly decreased odor compared to the control
2: Decreased odor compared to the control
1: Significantly decreased odor compared to the control
0: No odor

**[Table 1]**

| | Water-absorbent resin particles | | | Activated carbon | | | Silica | Heat release properties of the water-absorbent resin composition | | | | Deodorizing effect of the water-absorbent resin composition |
|---|---|---|---|---|---|---|---|---|---|---|---|---|
| | Physiological saline absorption amount (g/g) | Water-absorption rate (sec) by the Vortex method | Median particle size (µm) | Median particle size (AM) | BET specific surface area (m²/g) | Proportion (part(s) by mass) per 100 parts by mass of the water-absorbent resin particles | Proportion (part(s) by mass) per 100 parts by mass of the water-absorbent resin particles | Time (sec) required until +2.0°C | Heat Release rate (°C/sec) until +2.0°C | Temperature (°C) after 5 minutes | Heat Release temperature Δ (°C) | |
| Ex. 1 | 35 | 4 | 360 | 38 | 1619 | 0.3 | 0 | 6 | 0.33 | 29.5 | 4.6 | 1.0 |
| Ex. 2 | 41 | 29 | 312 | 38 | 1619 | 0.3 | 0 | 16 | 0.13 | 28.9 | 4.0 | 1.3 |
| Ex. 3 | 41 | 29 | 312 | 7 | 1495 | 0.3 | 0 | 14 | 0.14 | 29.0 | 4.1 | 1.3 |
| Ex. 4 | 41 | 29 | 312 | 38 | 1619 | 0.3 | 0.5 | 14 | 0.14 | 29.0 | 4.1 | 1.3 |
| Comp. Ex. 1 | 40 | 42 | 386 | 38 | 1619 | 0.3 | 0 | 22 | 0.09 | 28.4 | 3.5 | 2.0 |
| Comp. Ex. 2 | 42 | 7 | 66 | 38 | 1619 | 0.3 | 2.0 | 40 | 0.05 | 28.4 | 3.5 | 2.0 |
| Comp. Ex. 3 | 41 | 29 | 312 | - | - | 0 | 0 | 17 | 0.12 | 29.0 | 4.1 | 4.0 |

### Reference Signs List

1: burette unit
3: clamp
4: measurement unit
5: conduit
10a: water-absorbent resin particles
11: stand
13: measurement table
13a: through hole
15: nylon mesh sheet
21: burette tube
22: cock
23: rubber stopper
24: cock
25: air inlet tube
31: cylinder
32: polyamide mesh
33: weight

## Claims

1. A water-absorbent resin composition, comprising water-absorbent resin particles and an activated carbon disposed on surfaces of the water-absorbent resin particles,
wherein when, while stirring 50 g of physiological saline at 24.9°C at 600 rpm in a 300 mL thermally insulated container, 10 g of the water-absorbent resin composition is added and swollen, the water-absorbent resin composition has a heat release rate of 0.10°C/sec or more and 1.00°C/sec or less until temperature increases by 2°C.

2. The water-absorbent resin composition according to claim 1, wherein the activated carbon content is 0.05% by mass or more and 1% by mass or less.

3. The water-absorbent resin composition according to claim 1 or 2, wherein the activated carbon has a median particle size of 1 µm or more and 500 µm or less.

4. An absorbent material comprising the water-absorbent resin composition according to claim 1 or 2.

5. An absorbent article comprising the absorbent material according to claim 4.
